# EUROPEAN PATENT SPECIFICATION

(11) **EP 4 017 389 B1**
(45) Date of publication and mention of the grant of the patent: **12.03.2025**
(21) Application number: 20768171.9
(22) Date of filing: 21.08.2020
(51) Int. Cl.: A61B 18/04, A61B 1/00, A61B 1/307

(54) **DEVICE FOR TISSUE ABLATION**
VORRICHTUNG ZUR GEWEBEABLATION
DISPOSITIF POUR L'ABLATION DE TISSU

(30) Priority: 22.08.2019 US 201962890264 P
(43) Date of publication of application: 29.06.2022
(73) Proprietor: Boston Scientific Scimed, Inc., Maple Grove, MN 55311-1566 (US)
(72) Inventor: RAUNIYAR, Niraj, Prasad, Plymouth, MN 55446 (US); CARLSON, Steven, T., St. Paul, MN 55117 (US)
(74) Representative: Pfenning, Meinig & Partner mbB
(86) International application number: PCT/US2020/047442
(87) International publication number: WO 2021/035153

(56) References cited:
- EP-A2- 3 244 820
- US-A1- 2010 179 416
- US-A1- 2016 220 296
- US-A1- 2018 168 712

## Description

### TECHNICAL FIELD

The present disclosure relates generally to devices and systems for tissue ablation and, in particular, distal assemblies for vapor ablation devices.

### BACKGROUND

Certain medical conditions, such as conditions of the prostate, may be treated by ablation, including by vapor ablation. Such ablation may be performed using a device having a sheath that is inserted into a body lumen or otherwise into a body of a patient. In ablation procedures, visualization of the area being treated may assist in performing the procedure effectively. A user may also desire to utilize tools or deliver fluids during a procedure. However, components such as treatment elements (e.g., needles), visualization components, and/or working channels may increase a diameter of the sheath or overall device, thereby resulting in increased discomfort or recovery time for the patient.

The systems, devices, and methods of the current disclosure may rectify some of the deficiencies described above, and/or address other aspects of the prior art.

Document US 2018/168712 A1 describes systems and methods for prostate treatment. A vapor delivery needle is provided that may include any of a number of features. One feature of the energy delivery probe is that it can apply condensable vapor energy to tissue, such as a prostrate, to shrink, damage, denaturate the prostate. In some embodiments, the vapor delivery needle can be advanced a predetermined distance into the prostate by an actuation mechanism. The actuation mechanism can comprise, for example, a spring, or at least one magnet.

Document US 2016/220296 A1 describes a vapor delivery system and method that is adapted for treating prostate tissue. The vapor delivery system includes a vapor delivery needle configured to deliver condensable vapor energy to tissue. In one method, the vapor delivery system is advanced transurethrally into the patient to access the prostate tissue. The vapor delivery system includes a generator unit and an inductive heating system to produce a high quality vapor for delivery to tissue. Methods of use are also provided.

### SUMMARY

The invention is defined in the appended set of claims. In an example, a device for vapor ablation comprises a sheath extending from a proximal end to a distal portion. The sheath includes a lumen terminating distally at a lumen opening. The device further comprises a vapor delivery member received in the lumen. The vapor delivery member includes a channel configured to receive vapor and at least one aperture configured to deliver the vapor to a body tissue. The device further comprises an electrical component having a chip. The chip is disposed proximate to the distal lumen opening.

Any of the devices disclosed herein may have any of the following features. The electronic component may include at least one of a camera, an optical coherence tomography sensor, a spectrum analyzing sensor, or a force sensor. The sheath includes a working channel extending from the proximal end and terminating distally at a working channel opening. The electrical component is disposed radially between the working channel opening and the distal lumen opening. The working channel may have two convex sides, one concave side, and one straight side. The electrical component may have a face directed distally. The member may be configured to be transitioned from a first configuration to a second configuration. In the first configuration, the member may not extend out of the distal portion. In the second configuration, the member may extend out of an opening on a radially outer surface of the distal portion. When in the second configuration, a line extending normally from a surface of the electronic component may be transverse to the member. The electronic component may be disposed distally of the distal lumen opening. The sheath may include a plurality of working channels extending from the proximal end to the distal portion. The electrical component may be a first electrical component and the chip is a first chip. The device may further comprise a second electrical component having a second chip. The second chip may be disposed at the distal portion. Each of the first electrical component and the second electrical component may have a face directed distally. The sheath may have a substantially pear-shaped cross-section. The chip may be configured for spectral analysis.

In another example, a device for vapor ablation comprises a sheath extending from a proximal end to a distal portion and a lumen extending from the proximal end to a lumen opening at the distal portion. A vapor delivery member is disposed within the lumen. The device further a working channel extending from the proximal end to a working channel opening at the distal portion and an electrical component having a chip. The chip is disposed proximate to the lumen opening.

Any of the devices disclosed herein may have any of the following features. The electronic component may include at least one of a camera, an optical coherence tomography sensor, a spectrum analyzing sensor, or a force sensor. The electrical component may face at least partially distally.

In still another example, a device for vapor ablation comprises a sheath extending from a proximal end to a distal portion and a lumen extending from the proximal end to a lumen opening at the distal portion. A vapor delivery member is disposed within the lumen. The device further comprises a working channel extending from the proximal end to a working channel opening at the distal portion and an electrical component disposed radially between the lumen opening and the working channel opening.

Any of the devices disclosed herein may have any of the following features. The electronic component may include at least one of a camera, an optical coherence tomography sensor, a spectrum analyzing sensor, or a force sensor

It may be understood that both the foregoing general description and the following detailed description are exemplary and explanatory only and are not restrictive of the invention, as claimed. As used herein, the terms "comprises," "comprising," or any other variation thereof, are intended to cover a non-exclusive inclusion, such that a process, method, article, or apparatus that comprises a list of elements does not include only those elements, but may include other elements not expressly listed or inherent to such process, method, article, or apparatus. The term "exemplary" is used in the sense of "example," rather than "ideal." As used herein, the term "proximal" means a direction closer to an operator and the term "distal" means a direction further from an operator. Although vapor ablation is referenced herein, such references should not be construed as limiting. The examples disclosed herein may also be used with other types of ablation mechanisms (e.g., cryoablation, RF ablation, or other types of ablation) or with other devices not relating to ablation.

### BRIEF DESCRIPTION OF THE DRAWINGS

The accompanying drawings, which are incorporated in and constitute a part of this specification, illustrate examples of the present disclosure and together with the description, serve to explain the principles of the disclosure.
FIG. 1 is a cross-sectional view of a portion of an exemplary ablation device.
FIGS. 2A-2B are partial cross-sectional views of the exemplary ablation device of FIG. 1.
FIG. 3 is a perspective view of the exemplary ablation device of FIG. 1.
FIG. 4A is a partial cross-sectional view of another exemplary ablation device.
FIGS. 4B-4C are perspective views of the exemplary ablation device of FIG. 4A.

### DETAILED DESCRIPTION

A device for vapor ablation may include one or more electronic components including cameras, light sources, ultrasound sensors, or other sensors. Component(s) may be located near a distal tip of the device. The component(s) may be arranged so that the device has sufficient cross-sectional area available for a working channel. In one example, electronic component(s) may face at least partially distally. In another example, electronic component(s) may face at least partially proximally. Still further, in other examples, electronic component(s) may face at least partially radially inwardly or partially radially outwardly relative to a longitudinal axis of the distal tip. Electronic component(s) may face a combination of directions (e.g., partially distally/proximally and partially radially inwardly/outwardly. The examples described herein may be used in combination. For example, a device may have electronic components that face both distally and proximally.

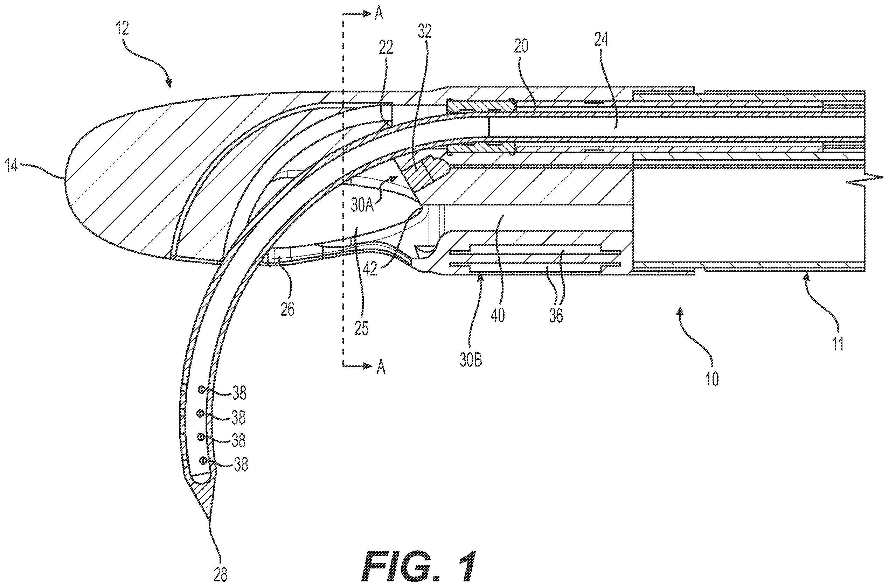

FIG. 1 shows a cross-section of an exemplary distal assembly of an ablation device 10. Ablation device 10 may include a shaft 11, which may be insertable into a body lumen of a patient or otherwise into a body of a patient (e.g., through a tissue of a patient, such as via a transperineal route). Shaft 11 may have a distal tip 12. Distal tip 12 may include a distal-most surface 14. Distal-most surface 14 may have an atraumatic shape (e.g., a rounded, bulbous shape). Distal tip 12 and/or distal-most surface 14 may be made of any suitable material, including a plastic material, which may be transparent (e.g., acoustically transparent). Distal tip 12 may alternatively be made of metal, resin, Ultem^{®}, polyurethane, or other materials, or a combination of materials.

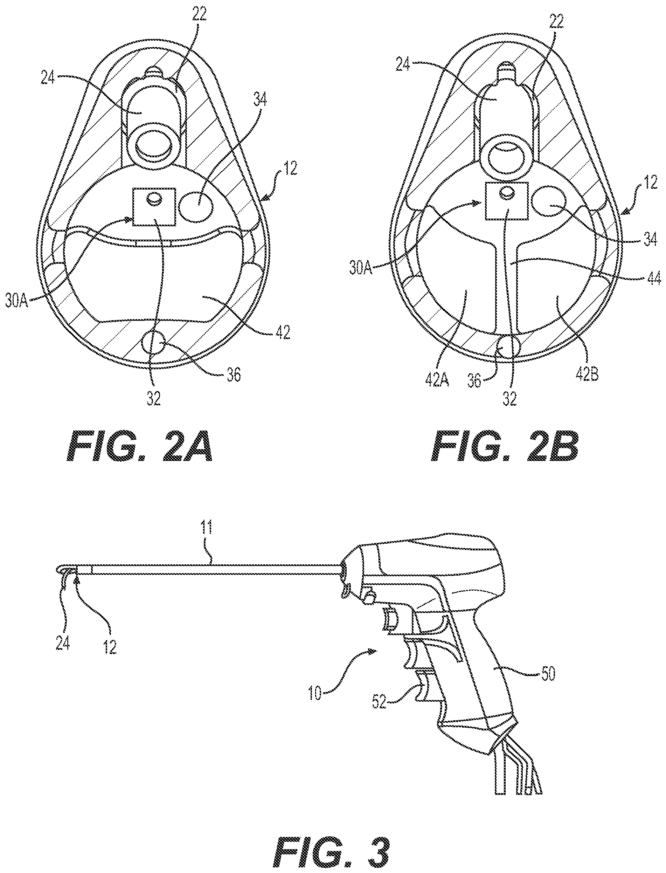

Device 10 may include a needle lumen 20 that extends from a proximal end of sheath 11 (not shown) to a distal opening 22. A needle 24 may be inserted into needle lumen 20. Needle 24 may be a member having a central lumen or channel extending from a proximal end of needle 24 (not shown) toward a distal tip 28 of needle 24, and a plurality of apertures 38 near distal tip 28. The plurality of apertures 38 may be configured to communicate the contents of the central lumen or channel (e.g., vapor, steam) to surrounding tissue into which distal tip 28 is positioned, received, or otherwise inserted. For example, the central lumen or channel of needle 24 may be configured to receive vapor therein (e.g., via a vapor generator fluidly coupled to the proximal end of needle 24) and to deliver the vapor to tissue via the apertures 38. Needle 24 may be configured to have a first, insertion configuration, in which needle 24 is contained, received, or otherwise positioned within lumen 20 (e.g., such that no portion of distal tip 28 extends radially outwardly of distal tip 12, relative to a longitudinal axis of distal tip 12). Needle 24 may have a second, treatment configuration (FIG. 1), in which distal tip 28 of needle 24 is extended out of distal opening 22 (e.g., and optionally, radially outwardly of distal tip 12, relative to the longitudinal axis of distal tip 12). In the treatment configuration, needle 24 may curve radially outward relative to the longitudinal axis of sheath 11. Needle 24 may extend through a gap 25 in distal tip 12 and through of a radial surface opening 26. In some aspects, distal tip 12 may include one or more surface features to guide needle 24 from the insertion configuration toward the treatment configuration. For example, one or more portions of distal tip 12 may be curved, slanted, or otherwise directed toward radial surface opening 26, such that, during advancement (e.g., distal advancement) of needle 24 through lumen 20 and distal opening 22, one or more portions (e.g., distal tip 28) of needle 24 may impact or otherwise interface with the one or more portions of distal tip 12 such that continued advancement of needle 24 may cause needle 24 to bend, deflect, or otherwise be guided toward (and through) radial surface opening 26. As will be described in further detail below, FIGS. 2A and 2B show cross-sectional proximal-facing views of distal tip 12, taken across cross-section A-A of FIG. 1, with needle 24 extending out of distal opening 22, according to a first and a second exemplary arrangement, respectively.

Distal tip 12 may also include one or more electronic components 30A, 30B, which, as shown in FIG. 1, may face distally and/or radially outward in a direction toward radial surface opening 26. A direction that a component 30A, 30B faces may include a direction in which a face of component 30A, 30B is directed. Although two electronic components 30A, 30B are shown in FIG. 1, it will be appreciated that any number of electronic components may be used. A direction and positioning of electronic components 30A, 30b as shown in FIG. 1 is merely exemplary. Electronic components 30A, 30B may face any suitable direction and may be positioned in any suitable manner. Electronic components 30A, 30B may assist in navigation of needle 24.

Electronic components 30A, 30B may utilize wafer-based chip technology. Chips of electronic components 30A, 30B may be located/disposed at or proximate to distal tip 12. For example, electronic components 30A, 30B may include a camera, a light source, ultrasound sensor, a heat-electronic component, optical coherence tomography sensor, spectrum analyzing sensor, force sensor, infrared sensor (such as near infrared sensor), ultraviolet sensor, pressure sensor, temperature sensor, chemical sensor, accelerometer, force sensor, magnetoresistance sensor, tunnel magnetoresistance sensor, or other type of sensor or electronics. Electronic components 30A, 30B may facilitate real-time procedural analyses and efficiencies and may be used in conjunction with various algorithms. Electronic components 30A, 30B may be used for subsurface visualization, procedural feedback, mapping of tissue and/or tagging of tissue. Different or similar types of sensors may be used in combination with one another. Where multiple electronic components 30A, 30B are used, all electronic components 30A, 30B may be utilized at once, or different electronic components 30A, 30B may be active depending on a type of procedure, a stage of a procedure, the findings during a procedure, etc. Electronic components 30A, 30B may be flexible, rigid, or semi-flexible. Additionally, one or more wires (not shown) may electronically couple electronic components 30A, 30B to one or more actuators 52 positioned on a handle 50 of sheath 11, as will be described in further detail below. For example, the one or more wires may extend proximally from electronic components 30A, 30B toward a proximal end of sheath 11 and may pass through a lumen in sheath 11 (e.g., other than a working channel 40 (described in further detail below) or the lumen 20).

As shown particularly in FIGS. 2A and 2B, electronic component 30A may include components such as camera 32 and light source 34. Although camera 32 and light source 34 are given as exemplary elements of electronic component 30A, it will be appreciated that a wide variety of types of devices may be used. Electronic component 30A may have a distal surface, and a line extending normally from the surface may be transverse to needle 24 when needle 24 is in the second, treatment configuration.

Camera 32 may be oriented so as to allow a user to view needle 24 as it is being extended out of opening 22 and/or radial surface opening 26 (e.g., as shown in FIGS. 1 and 2A-2B). Camera 32 may include any appropriate elements, such as lenses, imagers, circuitry, etc. Camera 32 may be capable of performing spectral analyses and/or of analyzing types of light outside of the visual light spectrum. For example, camera 32 may include an RBG-Ir chip or module or a chip capable of infrared spectrum analysis, which may provide spectral analysis and information on temperature gradient during a treatment. More than one camera 32 may be used. For example, different types of cameras 32 may be used, or multiple of the same type of camera 32 may be used to provide different views of a procedure site. A single camera 32 may also include multiple chips, including multiple types of chips capable of performing multiple kinds of analysis.

Light source 34 may be, for example, one or more light emitting diode (LED) devices (which may include multiple colors of light), fiber device, a fiber optic bundle (FOB) device, or another type of lighting device (e.g., lighting an entirety of distal tip 12 to create diffused light). Light source 34 may be a single light source or may include multiple light sources. Light source 34 may provide varying spectra of light to facilitate analysis at a procedure site. Light source 34 may be integrated into a unified structure with camera 32. Alternatively, light source 34 and camera 32 may be separate structures.

Light source 34 may be oriented in a same or similar direction to camera 32, as shown in FIGS. 2A and 2B. An orientation of light source 34 may facilitate visualization by camera 32. Camera 32 and light source 34 may be aligned at an approximately equal distance from a central longitudinal axis of sheath 11. Camera 32 and/or light source 34 may be positioned on a proximal side of gap 25 or elsewhere so that camera 32 and/or light source 34 are facing a medium (e.g., air) that is conducive to functions of camera 32 and/or light source 34. Camera 32 and/or light source 34 may face gap 25 or another open area or may be encapsulated with an appropriate material, such as the material of distal tip 12. For example, material of distal tip 12 may cover camera 32 and/or light source 34 and may provide light-guiding, deflecting, or other features. Camera 32 may be oriented so that opening 22 is in a direction toward a top of an image produced by camera 32 (and that may be shown on a display to a user), and radial surface opening 26 is in a direction toward a bottom of an image produced by camera 32. A display shown to a user may be a hands-free system such as an augmented reality system. Camera 32 and/or light source 34 may be between (along a radial direction) opening 22 and radial surface opening 26. Camera 32 and/or light source 34 may be closer to a central longitudinal axis of sheath 11 than is opening 22.

As shown in FIGS. 1, 2A, and 2B, electronic component 30B may include an ultrasound sensor 36. Ultrasound sensor 36 may face any direction, including a direction that is at least partially radially outward. An area of sheath 11 surrounding ultrasound sensor 36 may be acoustically transparent so as to transmit signals from ultrasound sensor 36 or another type of sensor, such as those discussed above.

Sheath 11 may also include a working channel 40. Working channel 40 may have a distal opening 42 (see FIG. 2A). Working channel 40 may be used to pass, for example, fluids or tools. Working channel 40 may be located (along a radial direction) between electronic component 30A and radial surface opening 26. Working channel 40 may be located toward a bottom of an image produced by camera 32 and displayed to a user. Working channel 40 may have a shape that is complementary to a shape of sheath 11 and/or other components of device 10 including electronic components 30A, 30B. For example, a cross-sectional shape of working channel 40 (see FIG. 2A) may have a shape that has two convex curved sides, one concave curved side, and one flat side, collectively defining a lumen of working channel 40. Electronic component 30A may be most proximate to a concave curved side of working channel 40.

As shown in FIG. 2B, working channel 40 may be divided by a divider 44 so that two sub-channels are formed. The sub-channels may terminate in two openings, 42A and 42B. Working channel 40 may be divided along an entire length of working channel 40. Alternatively, working channel 40 may only be divided along a portion (e.g., a distal portion, near openings 42A and 42B) of working channel 140. Openings 42A and 42B (and sub-channels of working channel 40) may have the same shape and/or size, as shown in FIG. 2B, or may have different shapes and/or sizes without departing from the scope of this disclosure. The sub-channels terminating in openings 42A and 42B may be used for the same purposes or for different purposes. For example, the sub-channel terminating in opening 42A may be used for one fluid, and the sub-channel terminating in opening 42B may be used for another fluid. Additionally or alternatively, the sub-channel terminating in opening 42A may be used for delivery of fluid source to a site in a subject's body, and the sub-channel terminating in opening 42B may be used for return of fluid from the subject's body (e.g., via suction). Additionally or alternatively, the sub-channels terminating in openings 42A and 42B may be used for different tools, or one of the sub-channels terminating in openings 42A and 42B may receive one or more tools, and the other may receive one or more fluids.

The configurations of working channel(s) 40 shown in FIGS. 1, 2A, and 2B are merely exemplary. Working channel(s) 40 may have any appropriate shape or features and may be positioned in a variety of ways with respect to other components of distal tip 10, such as electronic components 30A, 30B, and lumen 20.

Electronic component 30B may be positioned between working channel(s) 40 and an outer surface of sheath 11, as shown in FIGS. 1, 2A, and 2B. It will be appreciated that a placement of electronic components 30A, 30B are merely exemplary, and that electronic components 30A, 30B may be placed in any suitable position. Although electronic components 30A, 30B are shown as distal-facing, it will also be appreciated that electronic components 30A, 30B may face in any direction.

As shown in Figs. 2A and 2B, a cross-section of sheath 11 may have an approximately pear-shaped outermost surface. However, such a shape is merely exemplary and any other shape may be utilized. For example, sheath 11 may have a rounded cross section, ovular cross section, or any other suitable shape. A size of sheath 11 may be varies so as to be tailored to a patient, a treatment being performed, etc. A largest dimension of a cross section of sheath 11 may be smaller than 22 French.

As shown in FIG. 3, device 10 may have a handle 50 for gripping by a user during a procedure. Handle 50 may include mechanisms for controlling device 10, such as controls for needle 24. Handle 50 may also include an actuator 52, which may be an image or video capture button. Actuator 52 may work in conjunction with one or more of electrical components 30A, 30B to capture current readings by electrical component(s) 30A, 30B. For example, actuator 52 may capture a still image or moving video from camera 32. A position of actuator 52 shown in FIG. 3 is merely exemplary, and actuator 52 may be located in any suitable position, including on a separate generator or elsewhere on handle 50.

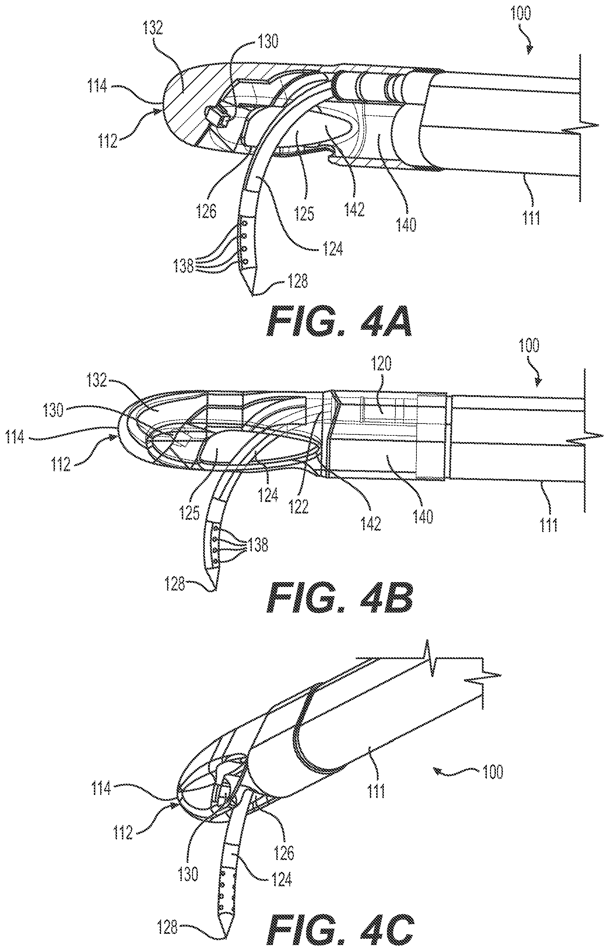

FIGS. 4A-4C show another example ablation device 100. FIG. 4A shows a partial cross-section of ablation device 100. FIG. 4B shows a perspective view of ablation device 100, with certain portions shown as being transparent. FIG. 4C shows another perspective view of ablation device 100 without transparency. Ablation device 100 may have any of the features of ablation device 10, described above, with similar features being designated with similar reference numerals plus "100". Ablation device 100 may include a shaft 111, which may be insertable into a body lumen of a patient or otherwise into a body of a patient (e.g., through a tissue of a patient, such as via a transperineal route). Shaft 111 may have a distal tip 112. Distal tip 112 may include a distal-most surface 114. Distal-most surface 114 may have an atraumatic shape (e.g., a rounded, bulbous shape). Distal tip 112 and/or distal-most surface 114 may be made of any suitable material, including a plastic material, which may be transparent. Distal tip 112 may alternatively be made of metal, resin, Ultem^{®}, polyurethane, or other materials, or a combination of materials.

Device 100 may include a needle lumen 120 (see FIG. 4B) that extends from a proximal end of sheath 111 (not shown) to a distal opening 122. A needle 124 (which may have any of the features of needle 24) may be inserted into needle lumen 120. Needle 124 may have a central lumen extending from a proximal end of needle toward a distal tip 128 of needle 124, and a plurality of apertures 138 near the distal tip. The plurality of apertures 138 may be configured to communicate the contents of the central lumen or channel (e.g., vapor, steam) to surrounding tissue into which distal tip 128 is positioned, received, or otherwise inserted. For example, the central lumen or channel of needle 124 may be configured to receive vapor therein (e.g., via a vapor generator fluidly coupled to the proximal end of needle 124) and to deliver the vapor to tissue via the apertures 138. Needle 124 may be configured to have a first, insertion configuration, in which needle 124 is contained, received, or otherwise positioned within lumen 120 (e.g., such that no portion of distal tip 128 extends radially outwardly of distal tip 112, relative to a longitudinal axis of distal tip 112). Needle 124 may have a second, treatment configuration (FIGS 4A-4C), in which distal tip 128 of needle 124 is extended out of distal opening 122 (e.g., and optionally, radially outwardly of distal tip 112, relative to the longitudinal axis of distal tip 112). In the treatment configuration, needle 124 may curve radially outward relative to the longitudinal axis of sheath 111. Needle 124 may extend through a gap 125 in distal tip 112 and through of a radial surface opening 126. In some aspects, distal tip 112 may include one or more surface features to guide needle 124 from the insertion configuration toward the treatment configuration. For example, one or more portions of distal tip 112 may be curved, slanted, or otherwise directed toward radial surface opening 126, such that, during advancement (e.g., distal advancement) of needle 124 through lumen 120 and distal opening 122, one or more portions (e.g., distal tip 128) of needle 124 may impact or otherwise interface with the one or more portions of distal tip 112 such that continued advancement of needle 124 may cause needle 124 to bend, deflect, or otherwise be guided toward (and through) radial surface opening 126.

Distal tip 112 may also include one or more electronic components 130. Electronic components 130 may have any of the features of electronic components 30A, 30B, described above. Ablation device 100 may include electronic components 130 positioned as shown and described with respect to FIGS. 1 and 2A-2B, although electronic components 130 are not separately shown in FIGS. 4A-4C. As shown in FIGS. 4A-4C, electronic components 130 may alternatively or additionally be positioned in/on a surface of an atraumatic tip 132 that is distal to openings 122, 126, and/or gap 125. Electronic components 130 may face proximally and may be angled in a radial direction toward opening 126. Such positioning may enable electronic component 130 to have any of the functionality of electronic component 30, as discussed above. Although FIGS. 4A-4C show one exemplary electronic component 130, it will be appreciated that any number of electronic components 130 may be used. For example, electronic components 130 may also be positioned similarly to electronic components 30A, 30B of FIGS. 1, 2A, and 2B.

Sheath 111 may also include a working channel 140 having a distal opening 142. Working channel 140 may have any of the features of working channel 40 and may include multiple sub-channels (see, e.g., FIG. 2B). Positioning electronic components 130 in/on a surface of atraumatic tip 132 may further facilitate increasing a diameter of working channel 140, as components need not be positioned near opening 142.

Use of electronic components, such as components 30A, 30B, 130, may facilitate aspects of ablation procedures. For example, if electronic component 30 or 130 has ultrasound or other types of imaging capability, electronic components 30, 30A, and/or 130 may assist in positioning a needle such as needle 24/124. Such functionality may be particularly useful where needle 24/124 is positionable at variable distances from openings 22/122 and 26/126. Additionally, one or more wires (not shown) may electronically couple electronic component 130 to one or more actuators (not shown), which may have any features of actuator 52, described above. For example, the one or more wires may extend proximally from electronic component 30 toward a proximal end of sheath 111 and may pass through a lumen in sheath 111 (e.g., other than working channel 140 or lumen 120).

While principles of the present disclosure are described herein with reference to illustrative examples for particular applications, it should be understood that the disclosure is not limited thereto. Accordingly, the invention is not to be considered as limited by the foregoing description.

## Claims

1. A device (10; 100) for vapor ablation, the device (10; 100) comprising:
a sheath (11; 110) extending from a proximal end to a distal portion, wherein the sheath (11; 110) includes a lumen (20; 120) terminating distally at a lumen opening (22; 122);
a vapor delivery member (24; 124) received in the lumen (20; 120), wherein the vapor delivery member (24; 124) includes a channel configured to receive vapor and at least one aperture (38; 138) configured to deliver the vapor to a body tissue; and
an electrical component (30A, 30B; 130) having a chip, wherein the chip is disposed proximate to the distal lumen opening (22; 122),
wherein the sheath (11;110) includes a working channel (40; 140) extending from the proximal end and terminating distally at a working channel opening (42; 142), and wherein the electrical component (30A, 30B; 130) is disposed radially between the working channel opening (42; 142) and the lumen opening (22;122).

2. The device (10; 100) of claim 1, wherein the electrical component (30A, 30B; 130) includes at least one of a camera (32), an optical coherence tomography sensor, a spectrum analyzing sensor, or a force sensor.

3. The device (10; 100) of any one of the preceding claims, the sheath (11; 111) has a distal tip (12; 112) including a distal-most surface (14; 114), wherein the electrical component (30A, 30B; 130) is located at the distal tip (12; 112).

4. The device (10; 100) of claim 3, wherein the electrical component (30A, 30B; 130) is encapsulated with a material of the distal tip (12).

5. The device (10; 100) of claim 3 or claim 4, wherein the working channel (40; 140) has two convex sides, one concave side, and one straight side.

6. The device (10; 100) of any one of the preceding claims, wherein the electrical component (30A, 30B; 130) has a face directed distally.

7. The device (10; 100) of any one of the preceding claims, wherein the vapor delivery member (24) is configured to be transitioned from a first configuration to a second configuration, wherein, in the first configuration, the vapor delivery member (24) does not extend out of the distal portion and wherein, in the second configuration, the vapor delivery member (24) extends out of a radial surface opening (26) on an outer surface of the distal portion.

8. The device (10; 100) of claim 7, wherein the radial surface opening (26) is on a radially outer surface of the distal portion.

9. The device (10; 100) of claim 7, wherein, when in the second configuration, a line extending normally from a surface of the electrical component (30A, 30B; 130) is transverse to the member (24).

10. The device (10; 100) of claim any one of the preceding claims, wherein the electrical component (30A, 30B; 130) is disposed distally of the distal lumen opening (22; 122).

11. The device (10; 100) of any one of the preceding claims, wherein the sheath (11; 110) includes a plurality of working channels (40; 140) extending from the proximal end to the distal portion.

12. The device (10; 100) of any one of the preceding claims, wherein the electrical component (30A, 30B; 130) is a first electrical component and the chip is a first chip, and wherein the device (10; 100) further comprises a second electrical component (30A, 30B; 130) having a second chip, wherein the second chip is disposed at the distal portion.

13. The device (10; 100) of claim 12, wherein each of the first electrical component and the second electrical component has a face directed distally.

14. The device (10; 100) of claim 1, wherein the sheath (11; 110) has a substantially pear-shaped cross-section.

15. The device (10; 100) of claim 1, wherein the chip is configured for spectral analysis.

## Patentansprüche

1. Vorrichtung (10; 100) zur Dampfablation, wobei die Vorrichtung (10; 100) umfasst:
eine Hülse (11; 110), die sich von einem proximalen Ende zu einem distalen Teil erstreckt, wobei die Hülse (11; 110) ein Lumen (20; 120) aufweist, das distal an einer Lumenöffnung (22; 122) endet;
ein Dampfzufuhrelement (24; 124), das in dem Lumen (20; 120) aufgenommen ist, wobei das Dampfzufuhrelement (24; 124) einen Kanal, der dazu eingerichtet ist, Dampf aufzunehmen, und mindestens eine Öffnung (38; 138) umfasst, die dazu eingerichtet ist, den Dampf an ein Körpergewebe abzugeben; und
eine elektrische Komponente (30A, 30B; 130) mit einem Chip, wobei der Chip in der Nähe der distalen Lumenöffnung (22; 122) angeordnet ist,
wobei die Hülse (11; 110) einen Arbeitskanal (40; 140) aufweist, der sich von dem proximalen Ende aus erstreckt und distal an einer Arbeitskanalöffnung (42; 142) endet, und wobei die elektrische Komponente (30A, 30B; 130) radial zwischen der Arbeitskanalöffnung (42; 142) und der Lumenöffnung (22; 122) angeordnet ist.

2. Vorrichtung (10; 100) nach Anspruch 1, wobei die elektrische Komponente (30A, 30B; 130) mindestens eines des Folgenden aufweist: eine Kamera (32), einen optischen Kohärenztomographiesensor, einen Spektrumanalysesensor oder einen Kraftsensor.

3. Vorrichtung (10; 100) nach einem der vorangehenden Ansprüche, wobei die Hülse (11; 111) eine distale Spitze (12; 112) mit einer distalsten Oberfläche (14; 114) aufweist, wobei die elektrische Komponente (30A, 30B; 130) an der distalen Spitze (12; 112) angeordnet ist.

4. Vorrichtung (10; 100) nach Anspruch 3, wobei die elektrische Komponente (30A, 30B; 130) mit einem Material der distalen Spitze (12) verkapselt ist.

5. Vorrichtung (10; 100) nach Anspruch 3 oder Anspruch 4, wobei der Arbeitskanal (40; 140) zwei konvexe Seiten, eine konkave Seite und eine gerade Seite aufweist.

6. Vorrichtung (10; 100) nach einem der vorangehenden Ansprüche, wobei die elektrische Komponente (30A, 30B; 130) eine nach distal gerichtete Fläche aufweist.

7. Vorrichtung (10; 100) nach einem der vorangehenden Ansprüche, wobei das Dampfzufuhrelement (24) dazu eingerichtet ist, um von einer ersten Konfiguration in eine zweite Konfiguration überführt werden zu können, wobei sich das Dampfzufuhrelement (24) in der ersten Konfiguration nicht aus dem distalen Teil heraus erstreckt und wobei sich das Dampfzufuhrelement (24) in der zweiten Konfiguration aus einer radialen Oberflächenöffnung (26) an einer äußeren Oberfläche des distalen Teils heraus erstreckt.

8. Vorrichtung (10; 100) nach Anspruch 7, wobei sich die radiale Oberflächenöffnung (26) an einer radial äußeren Oberfläche des distalen Teils befindet.

9. Vorrichtung (10; 100) nach Anspruch 7, wobei in der zweiten Konfiguration eine Linie, die sich senkrecht von einer Oberfläche der elektronischen Komponente (30A, 30B; 130) erstreckt, quer zu dem Element (24) verläuft.

10. Vorrichtung (10; 100) nach einem der vorangehenden Ansprüche, wobei die elektrische Komponente (30A, 30B; 130) distal von der distalen Lumenöffnung (22; 122) angeordnet ist.

11. Vorrichtung (10; 100) nach einem der vorangehenden Ansprüche, wobei die Hülse (11; 110) eine Vielzahl von Arbeitskanälen (40; 140) aufweist, die sich vom proximalen Ende zum distalen Teil erstrecken.

12. Vorrichtung (10; 100) nach einem der vorangehenden Ansprüche, wobei die elektrische Komponente (30A, 30B; 130) eine erste elektrische Komponente ist und der Chip ein erster Chip ist, und wobei die Vorrichtung (10; 100) ferner eine zweite elektrische Komponente (30A, 30B; 130) mit einem zweiten Chip umfasst, wobei der zweite Chip an dem distalen Teil angeordnet ist.

13. Vorrichtung (10; 100) nach Anspruch 12, wobei sowohl die erste elektronische Komponente als auch die zweite elektrische Komponente eine nach distal gerichtete Fläche aufweist.

14. Vorrichtung (10; 100) nach Anspruch 1, wobei die Hülse (11; 110) einen im Wesentlichen birnenförmigen Querschnitt aufweist.

15. Vorrichtung (10; 100) nach Anspruch 1, wobei der Chip zur Spektralanalyse eingerichtet ist.

## Revendications

1. Dispositif (10 ; 100) pour l'ablation à la vapeur, le dispositif (10 ; 100) comprenant :
une gaine (11 ; 110) qui est étendue depuis une extrémité proximale jusqu'à une partie distale, dans lequel la gaine (11 ; 110) inclut une lumière (20 ; 120) qui est terminée de façon distale au niveau d'une ouverture de lumière (22 ; 122) ;
un élément de délivrance de vapeur (24 ; 124) qui est reçu dans la lumière (20 ; 120), dans lequel l'élément de délivrance de vapeur (24 ; 124) inclut un canal configuré pour recevoir de la vapeur et au moins une ouverture (38 ; 138) configurée pour délivrer la vapeur sur un tissu du corps ; et
un composant électrique (30A, 30B ; 130) qui comporte une puce, dans lequel la puce est disposée à proximité de l'ouverture de lumière distale (22 ; 122),
dans lequel la gaine (11, 110) inclut un canal de travail (40 ; 140) qui est étendu depuis l'extrémité proximale et qui est terminé de façon distale au niveau d'une ouverture de canal de travail (42 ; 142), et dans lequel le composant électrique (30A, 30B ; 130) est disposé de façon radiale entre l'ouverture de canal de travail (42 ; 142) et l'ouverture de lumière (22 ; 122).

2. Dispositif (10 ; 100) selon la revendication 1, dans lequel le composant électrique (30A, 30B ; 130) inclut au moins un moyen parmi une caméra (32), un capteur de tomographie par cohérence optique, un capteur d'analyse de spectre et un capteur de force.

3. Dispositif (10 ; 100) selon l'une quelconque des revendications précédentes, dans lequel la gaine (11 ; 110) comporte une pointe distale (12 ; 112) qui inclut une surface la plus distale (14 ; 114), et dans lequel le composant électrique (30A, 30B ; 130) est localisé au niveau de la pointe distale (12 ; 112).

4. Dispositif (10 ; 100) selon la revendication 3, dans lequel le composant électrique (30A, 30B ; 130) est encapsulé avec un matériau de la pointe distale (12).

5. Dispositif (10 ; 100) selon la revendication 3 ou la revendication 4, dans lequel le canal de travail (40 ; 140) comporte deux côtés convexes, un seul côté concave et un seul côté rectiligne.

6. Dispositif (10 ; 100) selon l'une quelconque des revendications précédentes, dans lequel le composant électrique (30A, 30B ; 130) comporte une face dirigée de façon distale.

7. Dispositif (10 ; 100) selon l'une quelconque des revendications précédentes, dans lequel l'élément de délivrance de vapeur (24) est configuré pour subir une transition depuis une première configuration selon une seconde configuration, dans lequel, dans la première configuration, l'élément de délivrance de vapeur (24) n'est pas étendu hors de la partie distale et dans lequel, dans la seconde configuration, l'élément de délivrance de vapeur (24) est étendu hors d'une ouverture de surface radiale (26) sur une surface externe de la partie distale.

8. Dispositif (10 ; 100) selon la revendication 7, dans lequel l'ouverture de surface radiale (26) est sur une surface radialement externe de la partie distale.

9. Dispositif (10 ; 100) selon la revendication 7, dans lequel, dans la seconde configuration, une ligne qui est étendue perpendiculairement à une surface du composant électrique (30A, 30B ; 130) est transversale par rapport à l'élément (24).

10. Dispositif (10 ; 100) selon l'une quelconque des revendications précédentes, dans lequel le composant électrique (30A, 30B ; 130) est disposé de façon distale par rapport à l'ouverture de lumière distale (22 ; 122).

11. Dispositif (10 ; 100) selon l'une quelconque des revendications précédentes, dans lequel la gaine (11 ; 110) inclut une pluralité de canaux de travail (40 ; 140) qui sont étendus depuis l'extrémité proximale jusqu'à la partie distale.

12. Dispositif (10 ; 100) selon l'une quelconque des revendications précédentes, dans lequel le composant électrique (30A, 30B ; 130) est un premier composant électrique et la puce est une première puce, et dans lequel le dispositif (10 ; 100) comprend en outre un second composant électrique (30A, 30B ; 130) qui comporte une seconde puce, dans lequel la seconde puce est disposée au niveau de la partie distale.

13. Dispositif (10 ; 100) selon la revendication 12, dans lequel chaque composant électrique parmi le premier composant électrique et le second composant électrique comporte une face dirigée de façon distale.

14. Dispositif (10 ; 100) selon la revendication 1, dans lequel la gaine (11 ; 110) présente une aire en coupe transversale sensiblement en forme de poire.

15. Dispositif (10 ; 100) selon la revendication 1, dans lequel la puce est configurée pour l'analyse spectrale.
